(19) Europäisches Patentamt
European Patent Office
Office européen des brevets

(11) Publication number: 0 186 117 B1

(12) EUROPEAN PATENT SPECIFICATION

(45) Date of publication of patent specification: 22.11.90

(21) Application number: 85116161.2

(22) Date of filing: 18.12.85

(51) Int. Cl.[5]: C 07 C 45/54, C 07 C 49/813, C 07 C 45/46, C 07 C 49/403, C 07 C 205/06, C 07 C 255/50, C 07 C 253/00, C 07 C 67/14, C 07 C 323/50

# (54) Process for the production of acylated diketonic compounds.

(30) Priority: 20.12.84 US 683882
20.11.85 US 798842

(43) Date of publication of application:
02.07.86 Bulletin 86/27

(45) Publication of the grant of the patent:
22.11.90 Bulletin 90/47

(84) Designated Contracting States:
AT BE CH DE FR GB IT LI NL SE

(56) References cited:
EP-A-0 017 195
EP-A-0 090 262
EP-A-0 123 001
EP-A-0 162 336
EP-A-0 186 118

SYNTHESIS, December 1978, pages 925-927, Georg Thieme Publishers; A. AKHREM et al.: "A new simple synthesis of 2-acylcyclohexane-1,3-diones"

(73) Proprietor: STAUFFER CHEMICAL COMPANY
Westport Connecticut 06881 (US)

(72) Inventor: Heather, James Brian
2198 Lupine
Hercules Calif. 94547 (US)
Inventor: Milano, Pamela Denise
1500 Pine Street No. 23
Concord Calif. 94520 (US)

(74) Representative: Kraus, Walter, Dr. et al
Patentanwälte Kraus, Weisert & Partner
Thomas-Wimmer-Ring 15
D-8000 München 22 (DE)

EP 0 186 117 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background and Prior Art

This invention pertains to a process for the production of acylated diketonic compounds by rearrangement of corresponding enol esters.

The types of compounds which will be referred to hereinafter as acylated diketonic compounds have the general formula

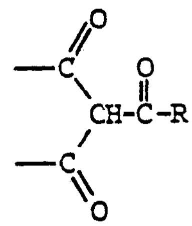

in which R is a alkyl, alkenyl, aryl, phenalkyl, or ether substituents, for instance those mentioned in the references described below. Compounds of this type have been described in a number of references as being useful, for instance, as chemical intermediates and/or pesticides. The rest of the molecule, which includes the diketonic group, has a generally cyclical structure. Most preferably the diketonic group includes a 5- to 6-member carbocyclic ring. Acylated diketonic compounds of this type have the general structure

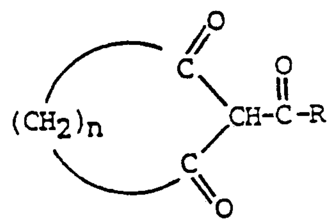

in which R is as defined above and n is 2 or 3. The ring may be unsubstituted or substituted at one or more positions by, for instance, alkyl, aryl, alkylene, etc. groups.

One method for production of acylated diketonic compounds is disclosed in European Patent Application, Publication No. 90262 and involves the reaction of an optionally substituted 1,3-cyclohexanedione with a substituted benzoyl cyanide. The reaction is carried out in the presence of zinc chloride and triethylamine. Such a process, however, has some drawbacks. Benzoyl cyanides are somewhat expensive reagents, and hydrogen cyanide is produced by this reaction in quantities of about one mole for each mole of acylated diketone. It would be desirable therefore to conduct the reaction using a less expensive and more readily available type of acylating agent which additionally did not produce such quantities of hydrogen cyanide. Benzoyl chlorides, for instance, are a relatively inexpensive and available form of acylating agent. However, benzoyl chlorides are stronger acylating agents than benzoyl cyanides and in the presence of the usual catalysts will tend not to acylate at the carbon atom between the two carbonyl groups, but rather directly attack one of the carbonyl groups itself, forming an enol ester of the type

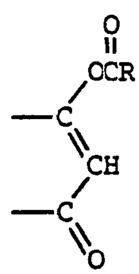

,

It is known from a number of references that acylated cyclic diketonic compounds may be produced from the corresponding enol esters by rearrangement;

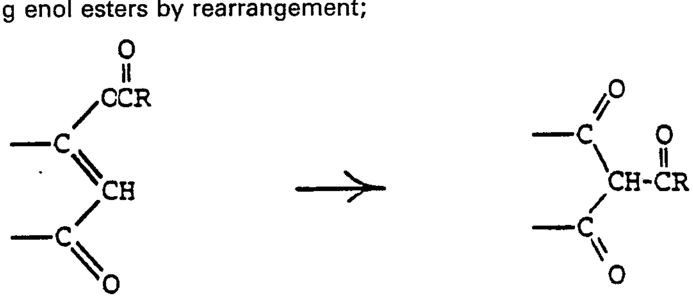

The references disclose several different types of acylated diketonic compounds and various catalysts or promoters for the rearrangement of enol esters to the acylated diketones.

For instance, Akhrem et al., *Synthesis*, p. 925—927 (1978) disclose the production of a number of acylated cyclohexanediones by reaction of 1,3-cyclohexanedione with an acylating agent (particularly an acyl halide) in two stages. In the first stage the acyl halide is reacted with the cyclohexanedione in the presence of pyridine to produce an enol ester, which is then converted to the acylated cyclohexanedione by rearrangement in the presence of a two-molar excess of aluminum chloride. Acrylating agents used in this work had the formula RCOCl in which R was various alkyl (e.g., methyl, ethyl, propyl), phenyl, substituted phenyl, benzyl, and substituted benzyl groups.

Tanabe et al., *Chem. Letters*, p. 53 (1982) describe work on production of 3-acyl-4-hydroxy-2-pyrones by acylation of pyrones with alkyl- or alkenyl-type acyl halides and rearrangement of the enol ester formed using a catalytic amount of 4-dimethylaminopyridine.

European Patent Application (Publication No.) 123001 discloses that other aminopyridine derivatives as well as certain N-alkylimidazole derivatives are suitable catalysts for rearrangement of enol esters to acylated cyclohexanediones having a 5-carboxylate substituent.

USSR Patent 784,195 discloses rearrangement of an enol ester to produce 2-oleoyl-cyclohexane-1,3-dione in the presence of molten sodium acetate at 160—170°C. European Patent Application, Publication No. 80301 discloses rearrangement of enol esters of 5-(polymethylphenyl)-1,3-cyclohexanediones to the corresponding acylated cyclohexanediones in the presence of a Lewis acid. Acylating agents used included anhydrides and acyl halides of the formula RCOCl in which R was alkyl, fluoroalkyl, alkenyl, alkynyl, or phenyl.

Summary of the Invention

This invention pertains to a process for producing an acylated cyclical diketonic compound by rearrangement of an enol ester in which the rearrangement is conducted in the presence of a cyanide source.

Detailed Description of the Invention

This invention concerns a process for producing an acylated diketonic compound by rearrangement of an enol ester according to the general reaction

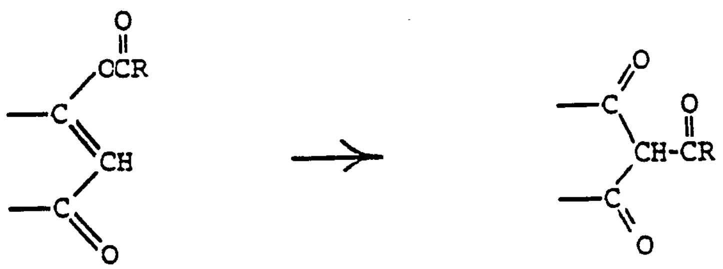

in which the rearrangement is conducted in the presence of a cyanide source.

More particularly, the rearrangement is conducted in the presence of either

(a) a catalytic amount of a cyanide source and a molar excess, with respect to the enol ester, of a moderate base, for instance, a tertiary amine, an alkali metal carbonate or an alkali metal phosphate; or

(b) a stoichiometric amount, with respect to the enol ester, of potassium cyanide or lithium cyanide, and a catalytic amount of a cyclical Crown ether or an acyclic analog thereof.

The products of this reaction have the general formula

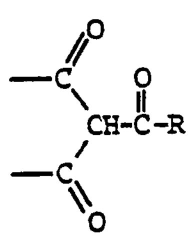

in which R may be variously alkyl, alkenyl, aryl (e.g., phenyl or substituted phenyl), phenalkyl (e.g. optionally substituted benzyl, phenethyl, etc.), or other substituents, for instance those mentioned in the references described above.

The remainder of the molecule includes a chain of atoms linking the carbon atoms of the two carbonyl groups so that the diketone is a cyclic compound. Most preferably the diketone is a carbocyclic compound. Preferred forms of diketones are cyclopentanediones and cyclohexanediones, which may be substituted at one or more positions on the ring by an alkyl, aryl or other substituent group. Most preferred are 1,3-cyclohexanediones, optionally substituted by one or more alkyl groups.

A particularly preferred class of products is that in which the diketone is a cyclohexanedione and the acylating group is a substituted benzoyl moiety. That is, R is substituted phenyl. In general, these compounds have the formula

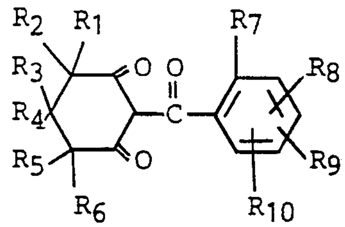

in which

$R_1$, $R_2$, $R_3$, $R_4$, $R_5$ and $R_6$ are independently hydrogen or $C_1$—$C_6$ alkyl (preferably $C_1$—$C_4$ alkyl) or $R_1$, $R_2$ or $R_3$ is

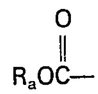

in which $R_a$ is $C_1$—$C_4$ alkyl; phenyl, optionally substituted by from 2 to 5 methyl groups; or in which $R_1$ and $R_2$, or $R_3$ and $R_4$, taken together are $C_3$—$C_6$ alkylene (such compounds have a spiro structure);

$R_7$ is halogen (chlorine, bromine, iodine or fluorine); cyano; $C_1$—$C_4$ alkyl; $C_1$—$C_4$ haloalkyl (preferably trifluoromethyl); $R_kS(O)_n$ in which $R_k$ is $C_1$—$C_4$ alkyl and n is preferably 0, 1 or 2; $C_1$—$C_4$ alkoxy (preferably methoxy) or nitro;

$R_8$, $R_9$ and $R_{10}$ independently are hydrogen or substituents including halogen; $C_1$—$C_4$ alkyl; $C_1$—$C_4$ alkoxy, trifluoromethoxy; cyano; nitro; $C_1$—$C_4$ haloalkyl; $C_1$—$C_4$ alkylthio; phenoxy; or substituted phenoxy in which substituent is halogen or halomethyl or both;

$R_bS(O)_n$ in which n is 0, 1 or 2; and $R_b$ is $C_1$—$C_4$ alkyl, $C_1$—$C_4$ haloalkyl, phenyl or benzyl,

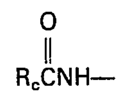

in which

$R_c$ is $C_1$—$C_4$ alkyl,

—$NR_dR_e$ in which $R_d$ and $R_e$ independently are hydrogen or $C_1$—$C_4$ alkyl;

$R_fC(O)$— in which $R_f$ is hydrogen, $C_1$—$C_4$ alkyl, $C_1$—$C_4$ haloalkyl or $C_1$—$C_4$ alkoxy;

$SO_2NR_gR_h$ in which $R_g$ and $R_h$ independently are hydrogen or $C_1$—$C_4$ alkyl; or

$R_8$ and $R_9$ taken together form a ring structure with two adjacent carbon atoms of the phenyl ring to which they are attached.

Compounds of this type, with various substituents on either or both of the cyclohexane or phenyl rings are disclosed in: European Patent Applications, Publication No. 90262, No. 135191, No. 137963 and US—P—4816066 (all relating to compounds which are herbicidal) and Japanese Patent Applications (Publication Nos.) 51/13750 and 51/13755 of Nippon Soda, K.K., which disclose some compounds of this type as intermediates for herbicides.

Some other compounds to which this process may be applied are disclosed in United States Patent No. 4822905 of David L. Lee t al, entitled "Certain 2-(2-Substituted Phenylacetyl)-1,3-Cyclohexanediones; and US—P—4681621 of David L. Lee et al, entitled "Certain 2-(2'-Substituted Benzoyl)-1,3-Cyclohexanediones", and compounds disclosed in literature mentioned under the heading "Background and Prior Art".

The rearrangement process of this invention is carried out in the presence of a cyanide source. The term "cyanide source" refers to a substance or substances which under the rearrangement conditions consists of or generates hydrogen cyanide and/or cyanide anion. There are two primary embodiments.

In one embodiment, the process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1—4 carbon atoms in the alkyl groups, such as acetone or methyl isobutyl ketone cyanohydrins; cyanohydrins of benzaldehyde or of $C_2$—$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid reaction and is inexpensive. It may be used in either liquid or gaseous form; when used as a gas it may be purchased from a commercial supplier or generated on-site by reaction of a metal cyanide with an acid. Among cyanohydrins the preferred cyanide source is acetone cyanohydrin.

In this embodiment, the cyanide source is used in an amount of to about 50 mole percent based on the enol ester. It may be used in as little as about 1 mole percent to produce an acceptable rate of reaction at

about 40°C on a small scale. Larger scale reactions give more reproducible results with slightly higher catalyst levels of about 2 mole percent. Generally about 1—10 mole % of the cyanide source is preferred.

In this embodiment the process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethyanolamine, and pyridine. Suitable inorganic bases include potassium carboante and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalyst are the Crown ethers.

A number of different solvents may be usable in this process, depending on the nature of the acid chloride or the acylated product. A preferred solvent for this reaction is 1,2-dichloroethane. Other solvents which may be employed, depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 80°C. In some cases, for instance when there is a possible problem of excessive by-product formation (for instance, when using an ortho-cyano benzoyl halide and an alkali metal cyanide or acetone cyanohydrin as the cyanide source) the temperature should be kept at about 40°C maximum.

In the second primary embodiment of this process, potassium or lithium cyanide serves as the cyanide source, but is used in a stoichiometric amount with respect to the enol ester, without using a separate base. The cyanide source is employed together with a catalytic amount of a phase transfer catalyst which is a Crown ether or an acyclic analog thereof.

The preferred cyanide source in this embodiment is potassium cyanide. The preferred Crown ether for this source is 18-Crown-6. Other hexadentate compounds such as cyclohexy-18-Crown-6, dibenzo-18-Crown-6, and the acyclic compound pentaethylene glycol dimethyl ether, would also be suitable.

For lithium cyanide, 15-Crown-5 is suitable.

In this embodiment, a separate base is not necessary, and is not employed.

This embodiment is suitable for production of compounds of the general type, but has been found particularly satisfactory for use where milder conditions are advantageous or necessary to minimize by-product formation, such as in the preparation of benzoylated cyclohexanediones having an ortho-cyano substituent on the phenyl ring. This embodiment of the process can be carried out at room temperature. Solvents similar to the first embodiment may be employed; acetonitrile is preferred.

The process to either embodiment may be carried out using the enol ester as the starting material, or with generation of the enol ester *in situ,* for instance by reaction of an acylating agent with a diketone. The term "enol ester" as used herein, refers to an enol ester of a carboxylic acid.

When the enol ester is utilized as a starting material in either embodiment of the process, it may be prepared by any of a number of known means, including acylation of a diketonic material with, for instance, an acyl halide.

The production of acylated diketones according to this invention, starting with acylating agents and diketones (for example, acyl halides such as substituted benzoyl halides and diketones such as cyclohexanediones) may be advantageously carried out with or without isolation of the intermediate enol ester. When carried out in two steps, the acyl halide or other acylating agent and the diketone are reacted in the presence of a moderate base such as triethylamine. The enol ester may be isolated from the resulting product mix by known techniques, for instance washing the resultant solution with acid and base, and with saturated sodium chloride solution, and drying. Such a technique is advantageous when a different solvent is preferred for the second step — the rearrangement of the enol ester to the acylated diketone. The dried enol ester may then be mixed with an appropriate solvent such as acetonitrile or 1,2-dichloroethane, and contacted with the appropriate amounts of cyanide source and either moderate base or Crown ether, according to which embodiment is used, at an appropriate temperature, to produce the final product.

Alternatively, the enol ester may be retained in the reaction product and the second stage may be carried out (using the same solvent by adding a cyanide source and additional base if necessary (in that embodiment), to produce the acylated diketone.

In another process variation, the acylated diketonic product may be obtained in one step via the *in situ* formation and rearrangement of the enol ester by reacting the acyl halide or other acylating agent with the diketone in the presence of an appropriate amount of cyanide source and a suitable amount of a moderate base or Crown ether, according to which embodiment is used.

Comparable yields can be obtained either with or without isolation of the enol ester.

The acylated diketonic product is obtained from this reaction in the form of its salt in the first described embodiment. The desired acylated diketone may be obtained by acdification and extraction with an appropriate solvent, if necessary. In some cases, the product may be contaminated with small amounts of

the carboxylic acid corresponding to the acyl halide; such by-products may be removed by contacting the acidified product solution with dilute aqueous sodium hydroxide or other suitable base, to form the salt of the acid. In the second embodiment the acylated diketone may be obtained per se.

The conduct of the process of this invention is illustrated by the following examples.

Example 1

Rearrangement of Enol Ester

A series of experiments was carried out on the production of 2-(2',3',4'-trichlorobenzoyl)-1,3-cyclohexanedione by rearrangement of its enol ester utilizing various cyanide sources and solvents. The general procedure was as follows: 3.0 grams (0.0094 mole) of the enol ester (prepared by reaction of 2,3,4-trichlorobenzoyl chloride with 1,3-cyclohexanedione in the presence of triethylamine, and isolated) was dissolved in 10 milliliters (ml) of the indicated solvent and 10 mole percent of the indicated catalyst and 140 mole percent of triethylamine (both quantities with respect to the enol ester) were added. The mixture was maintained at ambient temperature and reaction was allowed to proceed for 4—18 hours. The reaction mixture was diluted with water and the solvent was removed by distillation under reduced pressure. The resulting aqueous mixture was acidified to a pH of about 1 by the slow addition of 6N hydrochloric acid, with stirring. The resulting solids were collected by filtration and dried to constant weight at 75°C.

The yield of the crude acylated diketone product, uncorrected for purity of the starting materials, is given below in Table 1.

TABLE 1

| Catalyst | Solvent | Theoretical Yield, % | Product Purity, % |
|---|---|---|---|
| KCN | acetonitrile | 91.3 | 82.8 |
| KCN | acetonitrile | 91.0 | 81.9 |
| KCN | acetonitrile | 95.3 | 84.6 |
| KCN | 1,2-dichloroethane | 87.3 | 76.0 |
| KCN | 90% 1,2-dichloroethane/ 10% dimethylformamide | 86.0 | 75.7 |
| NaCN | 1,2-dichloroethane | 78.7 | 80.3 |
| acetone cyanohydrin | acetonitrile | 92.0 | 80.1 |

Example 2

Preparation of Acylated Diketone Without Isolation of Enol Ester

This example illustrates the process starting from an acyl halide and a diketone, in one step, without isolation of the intermediate enol ester. The procedure was as follows:

There were placed in a flask 3.0 g (0.027 mole) 1,3-cyclohexanedione, 15 ml of the indicated solvent and 10 mole percent (with respect to the intermediate enol ester) of sodium cyanide. The reaction mixture was blanketed with nitrogen and maintained at about room temperature. Then, 300 mole percent triethylamine (based on the enol ester) was added, with the mixture still being kept at room temperature. Then, 100 ml percent (with respect to the dione) of 2,3,4-trichlorobenzoyl chloride was added to the mixture. The mixture was maintained at ambient temperature and rection was allowed to proceed for about 24 hours. The product was recovered as in Example 1, yielding 8.04 g of crude product (93.2% of theoretical, uncorrected for purity of the starting materials).

Examples 3—6

A series of experiments was conducted similar to that described in Example 2, but using different catalysts and solvents. The same reactants were employed. All catalysts were used in an amount of 10 mole %, based on the intermediate enol ester. Table 2 contains the results of these experiments, with the yileds being uncorrected for purity of the starting materials.

TABLE 2

| Ex. No. | Catalyst | Solvent | Theoretical Yield, % | Product Purity, % |
|---|---|---|---|---|
| 3 | KCN | methylene chloride | 81.1 | 80.5 |
| 4 | KCN | 1,2-dichloroethane | 87.5 | 69.9 |
| 5 | acetone cyanohydrin | 1,2-dichloroethane | 90.7 | 82.6 |
| 6 | acetone cyanohydrin | toluene | 90.4 | 79.3 |

Example 7

This example also represents the conduct of the process without isolation of the intermediate enol ester.

In a flask there were placed 15 grams (0.13 mole) 1,3-cyclohexanedione, 75 ml 1,2-dichloroethane and 0.24 ml (2 mole percent based on the enol ester) acetone cyanohydrin. The materials were placed under a nitrogen blanket and the flask placed in an ice bath.

Then, there were added in succession, 54.36 ml (34.96 g, 0.39 mole) triethylamine and 32.86 g (0.13 mole) 2,3,4-trichlorobenzoyl chloride dissolved in 125 ml 1,2-dichloroethane. When the addition of both the amine and the benzoyl chloride had been completed, the temperature of the reaction mixture was raised to 40°C and the mixture allowed to react for 2 hours. At the end of this time, monitoring with a high pressure liquid chromatograph indicated 84.6 area percent of the desired product, with the majority of the remainder being unreacted cyclohexanedione.

The reaction mixture was then cooled and diluted with 100 ml water. The pH, which was 9.8, was adjusted to 2.8 by addition of 3 M sulfuric acid with an additional 100 ml 1,2-dichloroethane added during this step to redissolve solids which began to precipitate. The mixture was separated into aqueous and organic phases. The aqueous layer (about 200 ml) had a pH of 2.6.

The organic phase was washed with water and again phase separated (the aqueous phase had a pH of 4). The organic phase was then washed with 2 portions of 2.5 N aqueous sodium hydroxide and again phase separated after each wash. The aqueous phases had pH values of 10.7 and 12.8, respectively. The organic phase was again washed with 100 ml water.

All of the aqueous phases obtained from the separation steps above were combined and acidified with 3 M sulfuric acid. The pH value had been reduced to 2.1. The combined aqueous phases were kept at a low temperature in an ice bath. Solids precipitated from solution and were collected by filtration. The solids were dried to a constant weight in a vacuum oven. There was obtained 39.19 grams of the desired product, m.p. 150—151°C. The structure of the product was confirmed by high pressure liquid chromatographic analysis and comparison with a known sample.

Example 8

Production of 2-Propanoyl-1,3-cyclohexanedione

To a mixture of 3.0 g (0.027 mole) of 1,3-cyclohexanedione and 3.8 ml (0.027 mole) of triethylamide in 15 ml methylene chloride, there was added dropwise 2.3 ml (0.027 mole) of propionyl chloride with stirring and cooling in a room temperature water bath. After continued stirring at ambient temperature for about 4 hours, an additional 7.5 ml (0.054 mole) of triethylamine and 0.25 ml (10 mole percent with respect to enol ester) of acetone cyanohydrin were added. The mixture was stirred at ambient temperature overnight, and was then diluted with water and acidified with 6 N hydrochloric acid. The phases were separated, and the aqueous phase was extracted with methylene chloride. The combined organic phases were dried over anhydrous sodium sulfate and concentrated under reduced pressure to give 4.68 g of crude product as a mixture of solid and liquid. The crude product was dissolved in methylene chloride and was extracted with 2.5 N sodium hydroxide solution followed by water. The combined aqueous phases were acidified with 6 N hydrochloric acid and extracted with methylene chloride. The organic extract was dried over anhydrous sodium sulfate and concentrated under reduced pressure to give 3.83 g of oily product (84% of theoretical). Structure of the product was confirmed by infrared, nuclear magnetic resonance and mass spectroscopy.

Examples 9 and 10

These examples illustrate the production of the following compounds.

Example 9

2-(2'-Nitrobenzyl)-1,3-cyclohexanedione

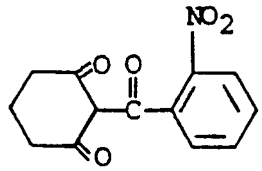

2-Nitrobenzoyl chloride (5.0 g, 0.027 mole) and cyclohexanedione (3.0 g, 0.027 mole) were dissolved in methylene chloride. Triethylamine (4.9 ml, 0.035 mole) was added dropwise and the resulting solution stirred for one hour. The solution was washed with 2N hydrochloric acid (2N HCl), water, 5% potassium carbonate solution and saturated sodium chloride solution, dried over anhydrous magnesium sulfate ($MgSO_4$) and concentrated under vacuum. The residue was dissolved in 20 ml acetonitrile. Triethylamine (1 equivalent) and potassium cyanide (40 mole %) were added and the solution stirred for one hour at room temperature. After dilution with ether, the solution was washed with 2N HCl and extracted with 5% potassium carbonate solution. The aqueous extract was acidified and ether was added. Filtration of the resulting mixture yielded 3.2 g of the desired compound (m.p. 132—135°C) which was identified by nuclear magnetic resonance, infrared and mass spectroscopy.

Example 10

2-(2'-Nitrobenzoyl)-5,5-dimethyl-1,3-cyclohexanedione

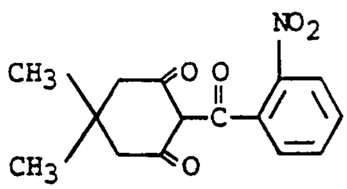

Triethylamine (3.4 ml, 0.025 mole) was added dropwise to a methylene chloride solution of 2-nitrobenzoyl chloride (3.5 g, 0.019 mole) and 5,5-dimethylcyclohexanedione (2.4 g, 0.019 mole). After stirring for one hour at room temperature an additional 3 equivalents of triethylamine and 0.4 ml acetone cyanohydrin were added. The solution was stirred for 2.5 hours, then washed with 2N HCl and extracted with 5% potassium carbonate solution. The basic extracts were acidified with 2N HCl and extracted with ether. The ether was washed with saturated sodium chloride solution, dried over anhydrous $MgSO_4$ and concentrated under vacuum. The residue was recrystallized from ethyl acetate yielding 2.0 g of the desired compound (m.p. 130—133°C) which was identified as such by nuclear magnetic resonance, infrared and mass spectroscopy.

Example 11

2-(2'-Cyanobenzoyl)-4,4-dimethyl-1,3-cyclohexanedione

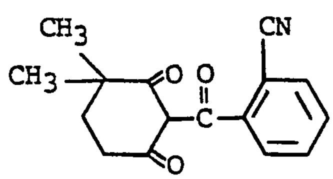

This example illustrates the production of the above-mentioned compound.

2-Cyanobenzoyl chloride (3.9 g, 0.024 mole) and 4,4-dimethyl-1,3-cyclohexanedione (3.3 g, 0.024 mole) were dissolved in 75 ml methylene chloride. Triethylamine (5.0 ml, 0.036 mole) was added dropwise and the resulting solution stirred for one and one-half hours at room temperature. The solution was washed with water, 2N HCl, 5% potassium carbonate solution (5% $K_2CO_3$) and saturated sodium chloride solution (brine), dried over anhydrous magnesium sulfate ($MgSO_4$) and concentrated under vacuum. The residue was dissolved in 20 ml acetonitrile. Triethylamine (4.4 ml, 0.032 mole) and acetone cyanohydrin (5 drops) were added and the solution stirred for two hours. After dilution with ether, the solution was washed with 2N HCl and extracted with 5% $K_2CO_3$. The aqueous extract was acidified with concentrated hydrochloric acid and extracted with ether. The ether was washed with water and brine, dried over $MgSO_4$ and concentrated under vacuum. The residue was purified by silica gel chromatography, yielding 1.2 g of a viscous oil which was identified as the desired compound by nuclear magnetic resonance, infrared and mass spectroscopy.

Example 12

2-(2'-Methylthiobenzoyl)-4,4,6-trimethyl-1,3-cyclohexanedione

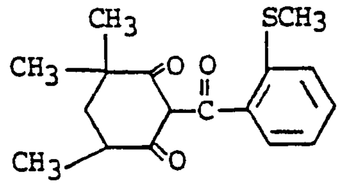

This example illustrates the production of the above-mentioned compound.

2-Methylthiobenzoyl (7.2 g, 0.039 mole) and 4,4,6-trimethylcyclohexanedione (5.0 g, 0.039 mole) were dissolved in methylene chloride. Triethylamine (7.0 ml, 0.050 mole) was added dropwise and the resulting solution stirred for one hour at room temperature. The solution was washed with water, 2N HCl, 5% $K_2CO_3$ and brine, dried over $MgSO_4$ and concentrated under vacuum. The residue was dissolved in 20 ml acetonitrile. Triethylamine (2.5 equivalents) and acetone cyanohydrin (0.4 ml) were added and the solution stirred for 45 minutes at room temperature. After dilution with ether, the solution was washed with 2N HCl and extracted with 5% $K_2CO_3$. The aqueous extract was acidified with concentrated hydrochloric acid and extracted with ether. The ether was washed with brine, dried over $MgSO_4$ and concentrated under vacuum. The residue was purified by trituration with ether, yielding 5.0 g of a viscous oil which was identified as the desired compound by nuclear magnetic resonance, infrared and mass spectroscopy (ms).

Example 13

2-(4'-Bromo-2'-trifluoromethylbenzoyl)-4,4,6-trimethyl-1,3-cyclohexanedione

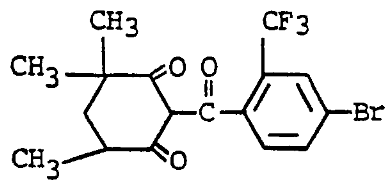

This example illustrates the production of the above mentioned compound.

4-Bromo-2-trifluoromethylbenzoyl chloride (4.3 g, 0.015 mole) and 4,4,6-trimethyl-1,3-cyclohexanedione (2.3 g, 0.015 mole) were dissolved in 100 ml methylene chloride. The solution was cooled with an ice bath and triethylamine (2.1 ml, 0.015 mole) in 10 ml methylene chloride was added dropwise. The ice bath was then removed and the resulting solution stirred for 30 minutes at room temperature. The solution was washed with 2N hydrochloric acid (2N HCl), 5% potassium carbonate solution (5% $K_2CO_3$) and saturated sodium chloride solution (brine), dried over anhydrous magnesium sulfate ($MgSO_4$) and concentrated under vacuum. The residue (5.1 g) was dissolved in 20 ml acetonitrile. Triethylamine (3.5 ml, 0.025 mole) and 0.4 ml acetone cyanohydrin were added and the solution stirred for two hours at room temperature while protected by a drying tube (calcium sulfate). After dilution with ether, the solution was washed with 2N HCl and extracted with 5% $K_2CO_3$. The aqueous extract was acidified with concentrated hydrochloric acid and extracted with ether. The ether was washed with brine, dried ($MgSO_4$) and concentrated under vacuum. The resulting oil was purified on a silica gel column (80:20:1 hexane:ethyl ethyl acetate:acetic acid — eluent), yielding 1.5 g of a viscous oil which was identified by nuclear magnetic resonance, infrared and mass spectroscopy.

Example 14

2-(4'-Chlorobenzoyl)-5,5-dimethyl-1,3-cyclohexanedione

This example illustrates the conduct of the process using hydrogen cyanide (generated by reaction of sodium cyanide with sulfuric acid) as the cyanide source.

5,5-Dimethylcyclohexane-1,3-dione (7.01 g, 0.05 mole) acetonitrile (80 ml) and trimethylamine (21 ml, 0.15 mole) were combined in a flask and placed under a nitrogen atmosphere. A solution of 4-chlorobenzoyl chloride (6.4 ml, 0.05 mole) in acetonitrile (20 ml) was added over 15 minutes while stirring and cooling with an ambient temperature water bath. In a separate reaction flask connected by a subsurface gas inlet tube, a solution of sulfuric acid (0.25 g, 0.0025 mole) in water (10 ml) was added over 10 minutes to a solution of sodium cyanide (0.25 g, 0.005 mole) in water (30 ml) at 85°C while stirring and slowly sweeping nitrogen through the secondary reactor and into the primary reactor. The primary reactor was then heated and stirred at 40°C for about 2 hours whereupon the reaction was completed.

The reaction mixture was diluted with 60 ml and slowly acidified with 40 ml 6N HCl with precipitation of the product. After stirring for about 5 minutes, the solid product was collected by filtration, washed with water, and dried to give 11.85 g (85.0% of theoretical yield) of off-white solids: m.p. 134—134.5°C.

Example 15

2-(4'-Chlorobenzoyl)-5,5-dimethyl-1,3-cyclohexanedione

This example illustrates the conduct of the process using a trilower alkyl silyl cyanide as the cyanide source.

5,5-Dimethylcyclohexane-1,3-dione (7.01 g, 0.05 mole) acetonitrile (80 ml) and triethylamine (21 ml, 0.15 mole) were combined in a flask and placed under a nitrogen atmosphere. A solution of 4-chlorobenzoyl chloride (6.4 ml, 0.05 mole) in acetonitrile (20 ml) was added over 15 minutes while stirring and cooling with an ambient temperature water bath. Trimethylsilyl cyanide (0.33 ml, 2.55 mmole) was added, and the reaction was heated and stirred at 40°C for 3 hours whereupon the reaction was complete.

The reaction mixture was diluted with 160 ml and slowly acidified with 40 ml 6N hydrochloric acid solution with precipitation of the product. After stirring for about 10 minutes, the product was collected by filtration and was washed with water and dried to afford 13.2 g (95.0% of theoretical yield) of off-white solids: m.p. 135—134.5°C.

Example 16

2-(2'-Cyanobenzoyl)-1,3-cyclohexanedione

This example illustrates the conduct of the second embodiment of the process, using a stoichiometric amount of potassium cyanide and a Crown ether.

In a flask were placed 1.2 g (0.005 mole) of the enol ester prepared by reaction of 1,3-cyclohexanedione with 2-cyanobenzoyl chloride, potassium cyanide (0.3 g, 0.005 mole), 18-Crown-6 (0.1 g, 0.0005 mole) and 10 ml acetonitrile. The mixture was stirred at room temperature for 30 minutes, then poured into 300 ml water. The pH was carefully adjusted to about 6 with concentrated hydrochloric acid; then the solution was extracted with 200 ml ethyl acetate. This in turn was extracted with 300 ml saturated aqueous solution of sodium bicarbonate. The bicarbonate extract was acidified (to pH about 3) with concentrated hydrochloric acid and extracted with 200 ml ethyl acetate. The resulting solution was dried over sodium sulfate and stripped, yielding 0.7 g (58% of theoretical yield) of the desdired product, an orange-brown oil. The structure was confirmed by infrared, nuclear magnetic resonance, and mass spectroscopy.

Example 17

2-(4'-Chlorobenzoyl)-5,5-dimethyl-1,3-cyclohexanedione

This example illustrates the conduct of the process using a metal carbonate as the base.

5,5-Dimethylcyclohexane-1,3-dione (3.50 g, 0.025 mole), potassium carbonate (10 g), potassium cyanide (0.2 g), and dimethylformamide (40 ml) were combined in a flask and placed under a nitrogen atmosphere. p-Chlorobenzoyl chloride (3.5 ml, 0.025 mole) was added dropwise. The mixture was stirred at 40°C for 3 hours and 70°C for 2 hours.

The reaction mixture was diluted with methylene chloride and acidified with 3N hydrochloric acid solution. The organic phase was washed with water and extracted with 2.5N sodium hydroxide solution. The basic extract was acidified with 3N hydrochloric acid solution. The precipitated product was collected by filtration, washed with water, and dried to afford 5.46 g (78.0% of theoretical yield) of crude product. Analysis of the product by HPLC (high performance liquid chromatography) showed 63% by weight of 2-(4'-chlorobenzoyl)-5,5-dimethyl-1,3-cyclohexanedione. p-Chlorobenzoic acid was the only major impurity.

**Claims**

1. A process for producing an acylated cyclical diketonic compound by rearrangement of the corresponding enol ester in which the rearrangement is conducted in the presence of either

(a) a catalytic amount of a cyanide source and a molar excess, with respect to the enol ester, of a moderate base; or

(b) a stoichiometric amount, with respect to the enol ester, of potassium cyanide or lithium cyanide, and a catalytic amount of a cyclical Crown ether or an acyclic analog thereof.

2. A process according to Claim 1 in which the rearrangement is conducted in the presence of a cyanide source, which is an alkali metal cyanide, zinc cyanide, a cyanohydrin of a methyl alkyl ketone having from 1—4 carbon atoms in the alkyl group; benzaldehyde cyanohydrin; a cyanohydrin of a $C_2$—$C_5$ alihatic aldehyde; a tri(lower alkyl) silyl cyanide or hydrogen cyanide, and a molar excess, with respect to the enol ester, of a moderate base.

3. A process according to Claim 2 in which the cyanide source is hydrogen cyanide.

4. A process according to Claim 2 in which the cyanide source is sodium cyanide.

5. A process according to Claim 2 in which the cyanide source is potassium cyanide.

6. A process according to Claim 2 in which the cyanide source is acetone cyanohydrin.

7. A process according to Claim 2 in which the cyanide source is used in an amount of from about 1 to about 10 mole percent, based on the enol ester.

8. A process according to Claim 2 in which the moderate base is a tertiary amine, an alkali metal carbonate or an alkali metal phosphate.

9. A process according to Claims 2—8 in which the moderate base is a trialkylamine.

10. A process according to Claims 2—9 in which the moderate base is triethylamine.

11. A process according to Claims 2—10 in which the base is used in an amount of from about 1 to about 4 moles per mol of enol ester.

12. A process according to Claim 1 in which the rearrangement is conducted in the presence of a stoichiometric amount, with respect to the enol ester, of potassium cyanide or lithium cyanide and a catalystic amount of a cyclical Crown ether or an acyclic analog thereof.

13. A process according to Claim 1 in which the acylated diketonic compound is a derivative of a cyclohexanedione.

14. A process according to Claim 1 in which the acylated diketonic compound is a substituted benzoyl derivative of a 1,3-cyclohexanedione.

15. A process according to Claim 1 in which the acylated diketonic compound has the formula

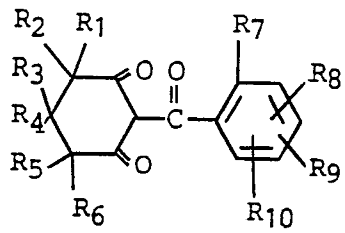

in which

$R_1$, $R_2$, $R_3$, $R_4$, $R_5$ and $R_6$ are independently hydrogen or $C_1$—$C_6$ alkyl or $R_1$, $R_2$ or $R_3$ is

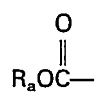

in which $R_a$ is $C_1$—$C_4$ alkyl, phenyl, optionally substituted by from 2 to 5 methyl groups; or in which $R_1$ and $R_2$ or $R_3$ and $R_4$, taken together are $C_3$—$C_6$ alkylene;

$R_7$ is halogen; cyano; $C_1$—$C_4$ alkyl; $C_1$—$C_4$ haloalkyl; $R_kS(O)_n$ in which $R_k$ is $C_1$—$C_4$ alkyl and n is 00, 1 or 2; $C_1$—$C_4$ alkoxy;

$R_8$, $R_9$ and $R_{10}$ independently are hydrogen; halogen; $C_1$—$C_4$ alkyl; $C_1$—$C_4$ alkoxy, trifluoromethoxy; cyano; nitro; $C_1$—$C_4$ haloalkyl; $C_1$—$C_4$ alkylthio; phenoxy; or substituted phenoxy in which the substituent halogen or halomethyl or both;

$R_bS(O)_n$ in which n is 0, 1 or 2; and $R_b$ is $C_1$—$C_4$ alkyl, $C_1$—$C_4$ haloalkyl, phenyl or benzyl,

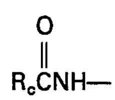

in which

$R_c$ is $C_1$—$C_4$ alkyl,

—$NR_dR_e$ in which $R_d$ and $R_e$ independently are hydrogen or $C_1$—$C_4$ alkyl;

$R_fC(O)$— in which $R_f$ is hydrogen, $C_1$—$C_4$ alkyl, $C_1$—$C_4$ haloalkyl or $C_1$—$C_4$ alkoxy;

$SO_2NR_gR_h$ in which $R_g$ and $R_h$ independently are hydrogen or $C_1$—$C_4$ alkyl; or

$R_8$ and $R_9$ taken together form a ring structure with two adjacent carbon atoms of the phenyl ring to which they are attached.

**Patentansprüche**

1. Verfahren zur Herstellung einer acylierten cyclischen Diketonverbindung durch Umlagerung des entsprechenden Enolesters, dadurch gekennzeichnet, daß die Umlagerung durchgeführt wird in Anwesenheit von entweder

(a) einer katalytischen Menge einer Cyanidquelle und einem molaren überschuß, bezogen auf den Enolester, einer milden Base oder

(b) einer stöchiometrischen Menge, bezogen auf den Enolester, von Kaliumcyanid oder Lithiumcyanid und einer katalytischen Menge eines cyclischen Crown-Ethers oder einem acyclischen Analogon davon.

2. Verfahren nach Anspruch 1, dadurch gekennzeichnet, daß die Umlagerung in Anwesenheit einer Cyanidquelle, welche ein Alkalimetallcyanid, Zinkcyanid, ein Cyanohydrin eines Methylalkylketons mit 1—4 Kohlenstoffatomen in der Alkylgruppe, Benzaldehydcyanohydrin, ein Cyanohydrin eines $C_2$—$C_5$ aliphatischen Aldehyds, ein Tri(niedrig-alkyl)silylcyanid oder Cyanwasserstoff ist und eines molaren Überschusses, bezogen auf den Enolester einer milden Base, durchgeführt wird.

3. Verfahren nach Anspruch 2, dadurch gekennzeichnet, daß die Cyanidquelle Cyanwassererstoff ist.

4. Verfahren nach Anspruch 2, dadurch gekennzeichent, daß die Cyanidquelle Natriumcyanid ist.

5. Verfahren nach Anspruch 2, dadurch gekennzeichent, daß die Cyanidquelle Kaliumcyanid ist.

6. Verfahren nach Anspruch 2, dadurch gekennzeichent, daß die Cyanidquelle Acetoncyanohydrin ist.

7. Verfahren nach Anspruch 2, dadurch gekennzeichent, daß die Cyanidquelle in einer Menge von etwa 1 bis etwa 10 mol-%, bezogen den Enolester verwendet wird.

8. Verfahren nach Anspruch 2, dadurch gekennzeichent, daß die milde Base ein tertiäres Amin, ein Alkalimetallcarbonat oder ein Alkalimetallphosphat ist.

9. Verfahren nach Anspruch 2 bis 8, dadurch gekennzeichent, daß die milde Base ein Trialkylamin ist.

10. Verfahren nach einem der Ansprüche 2 bis 9, dadurch gekennzeichnet, daß die milde Base Triethylamin ist.

11. Verfahren nach einem der Ansprüche 2 bis 10, dadurch gekennzeichent, daß die Base in einer Menge von etwa 1 bis etwa 4 mol pro Mol Enolester verwendet wird.

12. Verfahren nach Anspruch 1, dadurch gekennzeichent, daß die Umlagerung in Anwesenheit einer stöchiometrischen Menge, bezogen auf den Enolester, von Kaliumcyanid oder Lithiumcyanid und einer katalytischen Menge eines cyclischen Crown-Ethers oder eines acyclischen Analogons davon durchgeführt wird.

13. Verfahren nach Anspruch 1, dadurch gekennzeichent, daß die acylierte Diketonverbindung ein Derivat von Cyclohexandion ist.

14. Verfahren nach Anspruch 1, dadurch gekennzeichent, daß die acylierte Diketonverbindung ein substituiertes Benzoylderivat eines 1,3-Cyclohexandions ist.

15. Verfahren nach Anspruch 1, dadurch gekennzeichent, daß die acylierte Diketonverbindung die Formel

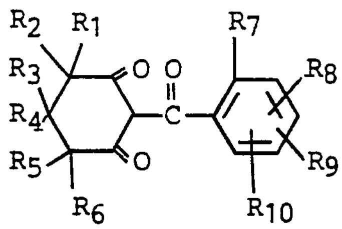

aufweist, worin

$R_1$, $R_2$, $R_3$, $R_4$, $R_5$ und $R_6$ unabhängig Wasserstoff oder $C_1$—$C_6$-Alkyl bedeuten oder $R_1$, $R_2$ oder $R_3$, $R_1$, $R_2$ oder $R_3$

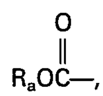

worin $R_a$ für $C_1$—$C_4$-Alkyl steht, Phenyl gegebenenfalls substituiert mit 2 bis 5 Methylgruppen bedeutet oder worin $R_1$ und $R_2$ oder $R_3$ und $R_4$ zusammen $C_3$—$C_6$-Alkylen bedeuten,

$R_7$ Halogen, Cyano, $C_1$—$C_4$-Alkyl, $C_1$—$C_4$-Haloalkyl, $R_kS(O)_n$, worin $R_k$ für $C_1$—$C_4$-Alkyl steht und n für 0, 1 oder 2 steht, $C_1$—$C_4$-Alkoxy bedeutet,

$R_8$, $R_9$ und $R_{10}$ unabhängig Wasserstoff, Halogen, $C_1$—$C_4$-Alkyl, $C_1$—$C_4$-Alkoxy, Trifluormethoxy, Cyano, Nitro, $C_1$—$C_4$-Haloalkyl, $C_1$—$C_4$-Alkylthio, Phenoxy oder substituiertes Phenoxy, worin der Substitutent für Halogen oder Halomethyl oder beides steht, $R_bS(O)$, worin n für 0, 1 oder 2 steht und $R_b$ für $C_1$—$C_4$-Alkyl, $C_1$—$C_4$-Haloalkyl, Phenyl oder Benzyl steht,

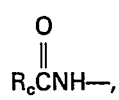

worin $R_c$ für $C_1$—$C_4$-Alkyl steht, —$NR_dR_e$, worin $R_d$ und $R_e$ unabhängig für Wasserstoff oder $C_1$—$C_4$-Alkyl stehen, $R_fC(O)$—, worin $R_f$ für Wasserstoff, $C_1$—$C_4$-Alkyl, $C_1$—$C_4$-Haloalkyl oder $C_1$—$C_4$-Alkoxy steht, $SO_2NR_gR_h$, worin $R_g$ und $R_h$ unabhängig für Wasserstoff oder $C_1$—$C_4$-Alkyl stehen, bedeuten oder worin $R_8$ und $R_9$ zusammen eine Ringstruktur mit zwei benachbarten Kohlenstoffatomen des Phenylrings, as den sie gebunden sind, bilden.

**Revendications**

1. Un procédé de production d'un composé dicétonique cyclique acylé par réarrangement de l'ester énolique correspondant dans lequel le réarrangement est mis en oeuvre en présence soit:

(a) d'une quantité catalytique d'une source de cyanure et d'un excès molaire par rapport à l'ester énolique d'une base modérée; ou

(b) d'un quantité stoechiométrique par rapport à l'ester énolique d'un cyanure de potassium ou d'un cyanure de lithium, et d'une quantité catalytique d'un éther couronne cyclique ou d'un de ses analogues acycliques.

2. Un procédé selon la revendication 1, dans lequel le réarrangement est mist oeuvre en présence d'une source de cyanure qui consiste en un cyanure de métal alcalin, un cyanure de zinc, une cyanohydrine de méthylalkylcétone dont le groupe alkyle comporte 1 à 4 atomes de carbone, une benzaldéhyde-cyanohydrine, un cyanohydrine d'un aldéhyde aliphatique en $C_2$—$C_5$, un cyanure de tri(alkyl inférieur)silyle ou un acide cyanhydrique, et un excès molaire par rapport à l'ester énolique d'une base moderée.

3. Un procédé selon la revendication 2, dans lequel la source de cyanure consiste en l'acide cyanhydrique.

4. Un procédé selon la revendication 2, dans lequel la source de cyanure est le cyanure de sodium.

5. Un procédé selon la revendication 2, dans lequel la source de cyanure est le cyanure de potassium.

6. Un procédé selon la revendication 2, dans lequel la source de cyanure est l'acétone cyanohydrine.

7. Un procédé selon la revendication 2, dans lequel la source de cyanure est utilisée en une quantité comprise entre environ 1 et environ 10 moles %, exprimée par rapport à l'ester énolique.

8. Un procédé selon la revendication 2, dans lequel la base modérée consiste en une amine tertiaire, un carbonate de métal alcalin ou un phosphate de métal alcalin.

9. Un procédé selon une des revendication 2 à 8, dans lequel la base modérée est un trialkylamine.

10. Un procédé selon une quelconque des revendications 2 à 9, dans lequel la base modérée est la triéthylamine.

11. Un procédé selon une quelconque des revendications 2 à 10, dans lequel la base est utilisée en une quantité comprise entre environ 1 et environ 4 moles part mole d'ester enolique.

12. Un procédé selon la revendication 1, dans lequel le réarrangement est mis en oeuvre en présence d'une quantité stoechiométrique par rapport à l'ester énolique de cyanure de potassium ou de cyanure de lithium et d'une quantité catalytique d'un éther couronne cyclique ou d'un de ses analogues acycliques.

13. Un procédé selon la revendication 1, dans lequel le composé dicétonique acylé est un dérivé de cyclohexanedione.

14. Un procédé selon la revendication 1, dans lequel composé dicétonique acylé est un dérivé benzoïque substitué d'une 1,3-cyclohexanedione.

15. Un procédé selon la revendications 1, dans lequel le composé dicétonique acylé présente la formule:

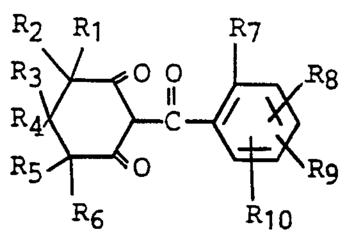

dans laquelle:

$R_1$, $R_2$, $R_3$, $R_4$, $R_5$ et $R_6$, indépendamment l'une de l'autre, consistent en un atome d'hydrogène ou un radical alkyle comprenant 1 à 6 atomes de carbone, ou $R_1$, $R_2$, $R_3$ sont des groupes $R_aOC(O)$— dans lesquels $R_a$ est un radical alkyle en $C_1$—$C_4$, un radical phényle éventuellement substitué par 2 à 5 groupes méthyles; ou dans lequel $R_1$ et $R_2$ ou $R_3$ et $R_4$, pris ensemble forment un radical alkylène en $C_3$—$C_6$;

$R_7$ est un halogène, un groupe cyano, alkyle en $C_1$—$C_4$, haloalkyle en $C_1$—$C_4$; $R_kS(O)_n$ dans lequel $R_k$ est un radical alkyle en $C_1$—$C_4$ et n est égal à 0, 1 ou 2, ou un radical alkoxy en $C_1$—$C_4$;

$R_8$, $R_9$ et $R_{10}$, indépendamment l'un de l'autre, consistent en un atome d'hydrogène ou d'halogène, un radical alkyle en $C_1$—$C_4$, alkoxy en $C_1$—$C_4$, trifluorométhoxy, cyano, nitro, haloalkyle en $C_1$—$C_4$, alkylthio en $C_1$—$C_4$, phénoxy, ou phénoxy substitué dans lequel le substituant est un atome d'halogène ou un groupe halométhyle ou les deux; $R_bS(O)_n$ dans lequel n est égal à 0, 1 ou 2, et $R_b$ et un radical alkyle en $C_1$—$C_4$, haloalkyle en $C_1$—$C_4$, phényle ou benzyle; $R_cC(O)NH$— dans lequel $R_c$ est un radical alkyle en $C_1$—$C_4$; —$NR_dR_e$ dans lequel $R_d$ et $R_e$, indépendamment l'un de l'autre, consistent en un atome d'ydrogène ou un radical alkyle en $C_1$—$C_4$; $R_fC(O)$— dans lequel $R_f$ est un atome d'hydrogène, un radical alkyle en $C_1$—$C_4$, haloalkyle en $C_1$—$C_4$ ou alkoxy en $C_1$—$C_4$; $SO_2NR_gR_h$ dans lequel $R_g$ et $R_h$, indépendamment l'une de l'autre, consistent en un atome d'hydrogène ou un radical alkyle en $C_1$—$C_4$; ou $R_8$ et $R_9$, pris ensemble, forment une structure cyclique avec deux atomes de carbone adjacents du noyau phényle auquel ils sont liés.